# FASCICULE DE BREVET EUROPEEN

(11) **EP 1 656 150 B1**
(45) Date de publication et mention de la délivrance du brevet: **20.12.2006**
(21) Numéro de dépôt: 04767850.3
(22) Date de dépôt: 23.06.2004
(51) Int. Cl.: A61K 35/74

(54) **UTILISATION DES BACILLI POUR LA PREVENTION DU SYNDROME DE COMPRESSION DES VERTEBRES**
VERWENDUNG VON BACILLI BAKTERIEN ZUR PRÄVENTION DES WIRBELKOMPRESSIONSSYNDROMS BEI SALMONIDEN
USE OF BACILLI BACTERIA FOR THE PREVENTION OF VERTEBRAL COMPRESSION SYNDROME IN SALMONIDS

(30) Priorité: 24.06.2003 FR 0350251
(43) Date de publication de la demande: 17.05.2006
(73) Titulaire: INSTITUT FRANCAIS DE RECHERCHE POUR L'EXPLOITATION DE LA MER (IFREMER), 92138 Issy-les-Moulineaux Cedex (FR); INSTITUT NATIONAL DE LA RECHERCHE AGRONOMIQUE (I.N.R.A.), 75341 Paris Cédéx 07 (FR)
(72) Inventeur: AUBIN, Joël, F-35650 Le Rheu (FR); LABBE, Laurent, F-29450 Sizun (FR); GATESOUPE, François-Joël, F-29217 Trebalu (FR); LEBRUN, Luc, F-29590 Pont De Buis (FR)
(74) Mandataire: Catherine, Alain
(86) Numéro de dépôt international: PCT/FR2004/050288
(87) Numéro de publication internationale: WO 2005/000327

(56) Documents cités:
- SILVERSTONE A.M. ET AL: "Spinal deformities in farmed Atlantic salmon." CANADIAN VETERINARY JOURNAL, (1 OCT 2002) 43/10 (782-784). REFS: 14 ISSN: 0008-5286 CODEN: CNVJA, 1 octobre 2002 (2002-10-01), XP0009027419
- GATESOUPE F J: "The use of probiotics in aquaculture" AQUACULTURE, vol. 180, no. 1-2, 1 octobre 1999 (1999-10-01), pages 147-165, XP0001179993 & ISSN: 0044-8486

## Description

La présente invention se rapporte au domaine de la prophylaxie du syndrome de compression des vertèbres chez les poissons appartenant à la famille des salmonidés.

On assiste aujourd'hui à une consommation humaine croissante des poissons appartenant à la famille des salmonidés, qui inclut notamment le saumon, la truite et l'omble. Afin de pouvoir proposer au consommateur final des poissons salmonidés à un coût modéré, ces poissons sont désormais produits de manière industrielle, par aquaculture, dans des élevages piscicoles. Ainsi, environ 53.000 tonnes de poissons frais issus de l'aquaculture ont été produits en France pendant l'année 2002. Parmi les salmonidés, la truite représente 77% des poissons frais produits dans les élevages piscicoles français.

Afin d'améliorer de manière constante les conditions économiques de la production de poissons frais d'aquaculture, les industriels ne cessent de faire progresser les conditions sanitaires et alimentaires des élevages, dans l'objectif de proposer au consommateur final des poissons frais de haute qualité sanitaire et gustative. Il est également tenu compte du rendement des élevages et les industriels cherchent à réduire, autant que possible, le taux de mortalité et l'incidence de pathologies diverses chez les poissons, particulièrement chez les poissons en phase de croissance.

Chez les salmonidés élevés en aquaculture, en particulier chez la truite arc-en-ciel, on observe une forte proportion d'individus atteints du syndrome de compression vertébrale, qui est une maladie du squelette entraînant (i) une malformation de certaines vertèbres et (ii) une soudure entre elles de certaines vertèbres, ces malformations squelettiques pouvant être réparties dans la région antérieure, la région pré-caudale et la région caudale de la colonne vertébrale du salmonidé.

Bien que le syndrome de compression vertébrale n'affecte pas la qualité sanitaire ou gustative du salmonidé, cette pathologie entraîne une déformation du squelette telle que le poisson est impropre à la présentation au consommateur final. Chez certains salmonidés, comme la truite arc-en-ciel, cette maladie peut atteindre jusqu'à 30% des poissons élevés dans des installations piscicoles. Le niveau d'incidence du syndrome de compression des vertèbres chez les salmonidés d'aquaculture réduit considérablement le rendement de production industrielle et a, en conséquence, une influence directe sur le prix de revient du poisson frais qui est commercialisé.

Dans la pratique, les pisciculteurs n'ont aujourd'hui d'autre choix que de procéder à une élimination, par simple détection à l'oeil nu, des individus atteints par le syndrome de compression des vertèbres. Ce tri est d'autant plus coûteux pour les élevages que la détection visuelle de ces malformations squelettiques n'est possible que lorsque les poissons sont en phase finale de croissance.

Aujourd'hui, le seul traitement connu permettant de réduire l'incidence du syndrome de compression des vertèbres consiste en un traitement antibiotique préventif à base du principe actif florphénicol. Un traitement d'une durée de 10 à 20 jours des truitelles avec cet antibiotique permet de réduire de plus de 60% la proportion des salmonidés atteints de cette maladie.

Toutefois, le recours à un antibiotique à titre préventif dans les élevages d'aquaculture présente de nombreux inconvénients. Notamment, un emploi extensif d'antibiotiques, en particulier du florphénicol, est susceptible de provoquer le développement et la dissémination d'une antibio-résistance, par exemple par transmission de plasmides, au sein de la flore microbienne présente dans les milieux d'élevage piscicole. Il va sans dire que le développement d'une résistance au florphénicol, qui est un antibiotique à large spectre d'activité, priverait l'industriel de tout recours prophylactique ou thérapeutique en cas d'infection bactérienne ultérieure de l'élevage. De plus, il ne peut être recouru à un traitement par un antibiotique qu'après un diagnostic vétérinaire préalable de l'élevage, puis une prescription spécifique par un praticien vétérinaire.

L'antibiotique reste présent, même à l'état de trace, dans la chair du poisson qui est ingérée par le consommateur final, ce qui est de nature à provoquer un phénomène d'antibio-résistance également dans la population humaine générale.

De plus, l'utilisation d'antibiotique, comme le florphénicol, peut entraîner un retard de croissance ainsi qu'une augmentation du taux de mortalité des alevins, par rapport aux alevins non traités.

Enfin, les réglementations encadrant les conditions d'élevage des poissons d'aquaculture sont de plus en plus strictes et pourraient en conséquence, dans un proche avenir, prohiber tout recours à l'utilisation d'un antibiotique.

Il serait donc souhaitable de trouver une alternative aux traitements prophylactiques actuels du syndrome de compression des vertèbres chez les salmonidés d'aquaculture, laquelle, notamment, éviterait tout recours à l'utilisation d'une composition pharmaceutique antibiotique et laquelle, de préférence, ne consisterait pas en un traitement de nature médicamenteuse.

De tels objectifs ont été atteints selon l'invention.

On a montré, selon l'invention, qu'une composition contenant des cellules d'au moins une souche de bactéries de la classe des *Bacilli* utilisée principalement comme additif alimentaire, permet de réduire l'incidence du syndrome de compression des vertèbres dans les élevages, avec au moins la même efficacité que l'antibiotique florphénicol.

De plus, on a montré selon l'invention que l'apport prophylactique d'une composition contenant des cellules d'au moins une souche de bactéries de la classe des *Bacilli* aux salmonidés d'élevage n'a aucune incidence sur le taux de survie des alevins, en comparaison avec un groupe d'alevins n'ayant pas reçu cet apport prophylactique.

En outre, l'apport prophylactique de bactéries de la classe des *Bacilli* n'entraîne aucune réduction dans la croissance des salmonidés d'aquaculture.

La présente invention a pour objet l'utilisation de cellules d'au moins une souche de bactéries de la classe des *Bacilli* pour la fabrication d'une composition destinée à prévenir le syndrome de compression des vertèbres chez un poisson appartenant à la famille des salmonidés.

Le syndrome de compression des vertèbres, qui peut être détecté de manière visuelle du fait des malformations apparentes des salmonidés en phase finale de croissance, consiste en deux types d'altérations du squelette, respectivement (i) une réduction de la largeur de certaines vertèbres par rapport à la normale et (ii) une soudure entre certaines vertèbres, dans une ou plusieurs régions de la colonne vertébrale, ces altérations squelettiques pouvant être détectées et visualisées par radiographie aux rayons X.

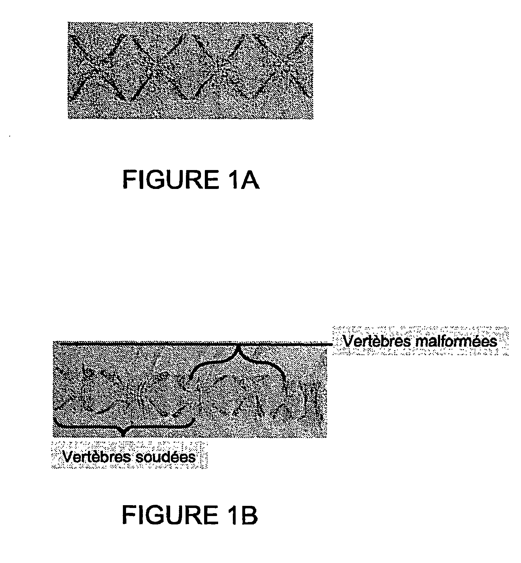

La figure **1A** représente un cliché radiographique aux rayons X d'une partie de la colonne vertébrale d'un salmonidé qui a eu une croissance squelettique normale.

La figure **1B** représente un cliché radiographique aux rayons X d'une partie de la colonne vertébrale d'un salmonidé atteint du syndrome de compression des vertèbres.

Comme on peut le voir sur la figure 1, le syndrome de compression des vertèbres entraîne une réduction de la largeur de la vertèbre, particulièrement pour les vertèbres qui sont soudées les unes aux autres. Selon l'intensité du syndrome de compression des vertèbres, c'est-à-dire selon le nombre de vertèbres atteintes, cette maladie induit une réduction globale plus ou moins grande de la longueur du poisson et, selon les zones de la colonne vertébrale qui sont affectées, également une distorsion de la croissance. De plus, ces anomalies squelettiques s'accompagnent d'une modification de la structure osseuse. En particulier, les vertèbres soudées présentent une compacité (surface osseuse/surface totale) significativement supérieure aux vertèbres des poissons non atteints.

Les malformations squelettiques des salmonidés atteints du syndrome de compression des vertèbres sont fréquentes essentiellement dans trois régions de la colonne vertébrale : (i) une région antérieure entre la vertèbre V8 et la vertèbre V21, (ii) une région pré-caudale entre la vertèbre V31 et la vertèbre V40 et (iii) une région caudale entre la vertèbre V51 et la vertèbre V61.

Cette troisième région squelettique, qui se situe dans le complexe urophore, à proximité de la nageoire caudale, présente surtout des vertèbres malformées mais très peu de vertèbres soudées.

Par « salmonidé », on entend selon l'invention des poissons osseux téléostéens des genres Oncorhynchus, Salmo, Salvenius, Hucho, Thymallus, Coregonus qui possèdent en commun de nombreuses caractéristiques anatomiques et physiologiques, telles que notamment un système digestif identique comprenant un oesophage, un estomac, des caeca pyloriques et un intestin court. Egalement, tous les salmonidés possèdent, entre les nageoires dorsales et caudales, une petite nageoire dite « nageoire adipeuse ».

Les caractéristiques anatomiques et physiologiques communes du système digestif de tous les salmonidés permettent de transposer aisément d'une espèce de salmonidé à l'autre un traitement prophylactique efficace à base de cellules de *Bacilli* qui entrent en contact en premier lieu avec les différentes parties du système digestif et qui sont en conséquence biologiquement actives dans cette partie de l'organisme.

Par « salmonidé », on entend selon l'invention les poissons téléostéens de la famille du saumon, tels que les saumons, les truites, les ombles, les ombres et les corégones.

Font partie du type saumon les poissons des espèces *Salmo salar L, , Oncorhynchus tshawytscha* (saumon *chinook*), *Oncorhynchus gorbuscha* (saumon rose), *Oncorhynchus nerka* (saumon *kokanee*), *Oncorhynchus keta* (saumon *keta*) et *Oncorhynchus kisutch* (saumon *coho*).

Font également partie des salmonidés les diverses truites, y compris *Salmo trutta L.* (truite de mer), *Salmo fario* (truite de rivières), *Salmo lacustris* (truite de lac), *Cristivomer namaycush* (truite canadienne), ou *Oncorhynchus mykiss* (truite arc-en-ciel) *Oncorhynchus clarki* (truite fardée).

Font également partie des salmonidés les poissons de type omble tels que *Salvelinus alpinus* (omble chevalier) ou encore *Salvelinus fontinalis* (saumon de fontaine) .

Font également partie des salmonidés les poissons du type ombre tels que *Thymallus thymallus* (ombre de rivière).

Font également partie des salmonidés les poissons du type corégone, tels que *Coregonus fera* (corégone fera), *Coregonus hiemalis* (gravenche), *Coregonus wartmanni* (lavaret), *Coregonus macrophthalmus* (bondelle), *Coregonus coregonus schinzi* (palée). Font également partie des salmonidés les poissons du type huchon *Hucho hocho.*

De préférence, on utilise les cellules d'au moins une souche de bactéries de la classe des *Bacilli* pour la fabrication d'une composition qui comprend de 10³ à 10⁸ unités formant des colonies (cfu) de ladite bactérie de la classe des *Bacilli* par gramme de la composition.

De préférence, on utilise la composition prophylactique telle que définie ci-dessus dans des quantités correspondant à un nombre d'unités formant des colonies (cfu) comprises entre 10⁹ et 10¹³ cfu par tonne de salmonidés par jour.

Par *« Bacilli »,* on entend selon l'invention les bactéries décrites dans le manuel de Bergey, et appartenant à la classe III du phylum des Firmicutes (Taxonomic outline of the procaryotes, Bergey's Manual of Systematic Bacteriology, 2^{nd} edition, July 2002, Georges M. Garrity, Kristin L. Johnson, Julia Bell and Denise B. Searles, Springer-Verlag, New York, pages 153 à 181).

Plus spécifiquement, par *« Bacilli* », on entend selon l'invention des bactéries du groupe *Bacillus-Lactobacillus-Streptococcus* (Tax ID 91061, NCBI, http://www.ncbi.nlm.nih.gov/Taxonomy/taxonomyhome.html). Sont préférées les bactéries des familles *Bacillaceae, Lactobacillaceae, Carnobacteriaceae, Enterococcaceae, Leuconostocaceae,* et *Streptococcacea.* Préférentiellement, on peut utiliser les souches *Bacillus subtilis* ATCC 6051, *Bacillus cereus* ATCC 14579 , *Bacillus megaterium* ATCC 14581, *Bacillus polymyxa* ATCC 842, *Bacillus licheniformis* ATCC 14580 , *Lactobacillus rhamnosus* ATCC 7469, *Lactobacillus plantarum* ATCC 14917, *Lactobacillus casei* ATCC 393, *Lactobacillus acidophilus* ATCC 4356, *Lactobacillus johnsonii* ATCC 33200, *Lactobacillus helveticus* ATCC 15009, *Lactobacillus bulgaricus* ATCC 11842, *Lactobacillus fructivorans* ATCC 8288, *Lactobacillus brevis* ATCC 14869, *Pediococcus acidilactici* DSM 20284, *Pediococcus pentosaceus* ATCC 33316, *Carnobacterium divergens* ATCC 35677, *Carnobacterium inhibens* CCUG 31728, *Enterococcus faecium* ATCC 19434, *Leuconostoc cremoris* ATCC 19254, *Weissella hellenica* ATCC 51523, *Streptococcus thermophilus* ATCC 19258, *Lactococcus lactis* ATCC 19435.

Une composition probiotique prophylactique utilisée selon l'invention peut comprendre des cellules d'une seule souche de bactéries de la famille des *Lactobacillaceae* telles que définies ci-dessus, ou encore plusieurs souches de ces bactéries, en association.

Lorsque plusieurs souches de bactéries de la famille des *Lactobacillaceae* sont présentes en association dans une composition prophylactique selon l'invention, sont combinées préférentiellement au plus 2, 3, 4 ou 5 souches distinctes, appartenant indifféremment aux genres *Bacillus, Lactobacillus, Pediococcus, Camobacterium, Enterococcus, Leuconostoc, Weissella, Streptococcus, Lactococcus.*

Préférentiellement, pour la fabrication d'une composition prophylactique selon l'invention, on utilise des cellules d'une souche de bactéries de la famille des *lactobacillaceae* choisie parmi *Lactobacillus rhamnosus* CNCM MA 543/2B, ou *Lactobacillus plantarum* CNCM MA 18/5U ou *Bacillus subtilis* R0179.

De manière particulièrement préférée, on utilise une souche de *Pediococcus acidolactici,* telle que la souche de *Pediococcus acidolactici* déposée à la Collection Nationale de Cultures de Microorganismes (CNCM) de l'Institut Pasteur de Paris, sous le numéro MA 18 5 M.

On peut également utiliser les souches *Lactobacillus rhamnosus* ATCC 53103, *Weissella hellenica* JCM10103, *Bacillus cereus* ATCC 14893, *Bacillus cereus* NCIB 40112.

Préférentiellement, la composition prophylactique utilisée selon l'invention comprend de 10⁴ à 10⁷ unités formant des colonies (cfu) de ladite bactérie de la classe des Bacilli, par gramme de la composition.

De préférence, le poisson appartenant à la famille des salmonidés est choisi parmi les saumons, les truites, les ombles, l'ombre, et les Coregones.

Selon un premier mode de réalisation préféré, la composition prophylactique utilisée selon l'invention consiste en une composition d'additif alimentaire destinée aux poissons appartenant à la famille des salmonidés.

L'invention a aussi pour objet un procédé pour la fabrication d'une composition d'additif alimentaire pour poissons salmonidés caractérisé en ce qu'il comprend une étape dans laquelle on incorpore des cellules d'une souche ou de plusieurs souches de bactérie de la classe des *Bacilli* à un matériau support physiologiquement compatible pour l'alimentation.

Dans ce premier mode de réalisation, le supplément ou l'additif alimentaire comprend les cellules de la ou des souche(s) de bactéries appartenant à la classe des *Bacilli* incorporées dans un support, de préférence sous la forme de particules, en particulier de granulés. Le support dans lequel sont incorporées les bactéries peut être de diverses natures. Par exemple, ledit support peut être constitué exclusivement, ou constitué essentiellement, d'amidon, y compris l'amidon de riz ou de maïs qui a été mélangé avec les cellules bactériennes avant sa transformation en particules, par exemple en granulés.

Le support dans lequel sont incorporées les cellules bactériennes peut également être constitué exclusivement, ou constitué essentiellement de β-cyclodextrine, la β-cyclodextrine étant alors mélangée avec les cellules bactériennes préalablement à une étape de séchage, puis de fabrication de particules, en particulier de granulés. La fabrication d'un additif alimentaire pour poissons comprenant un support à base de β-cyclodextrine, ainsi que sa fabrication sous la forme de granulés, est décrit notamment dans le brevet américain n°US 5,229,146.

Par exemple, pour la fabrication d'une composition alimentaire, aussi désignée additif alimentaire, selon l'invention, l'homme du métier peut mélanger la β-cyclodextrine avec une quantité appropriée de cellules bactériennes, de telle manière à obtenir une composition comprenant de 10³ à 10⁸ unités formant des colonies de ladite ou desdites bactérie(s) par gramme de la composition finale contenant la β-cyclodextrine, puis soumettre la composition ainsi obtenue à une étape de séchage, avant la fabrication de particules, en particulier de granulés, selon des méthodes conventionnelles.

Préférentiellement, le support peut être composé de carbonate de calcium, le cas échéant en combinaison avec du lactose.

Le support utilisé pour la fabrication d'une composition prophylactique d'additif alimentaire selon l'invention peut également être exclusivement constitué, ou essentiellement constitué, de lactose, le cas échéant en combinaison avec du silico-aluminate de sodium, combinaison à laquelle on ajoute le milieu de culture de la souche ou du mélange de souches de *Lactobacillaceae,* qui a été préalablement soumis à une étape de séchage.

Selon un autre aspect, pour fabriquer une composition prophylactique alimentaire selon l'invention, l'homme du métier peut utiliser des supports commerciaux, tels que les compositions de support « Ecoweaner® », « Ecostart® » commercialisés par la Société BIOMAR S.A. (France), ou encore les compositions « Néostart® 7 », « Néoprima® », « Néoextra® », « Néoultra® », « Néonova® », « Néoastra® » ou « Néosupra® », commercialisés par la Société LE GOUESSANT, (France) ou encore les compositions de support « Nutra HP®», « Classic® », « Select® » « Royal Optima® » commercialisés par la Société TROUW, (France).

Selon cet autre aspect, l'homme du métier ajoute à un poids donné de l'une de ces compositions commerciales, de préférence sous la forme d'un milieu de culture pour bactéries et contenant lesdites cellules de *Bacilli* à une valeur de cfu connue, en une quantité choisie de manière à obtenir une composition de mélange final à une concentration choisi, de 10⁴ à 10⁷ unités formant des colonies (cfu), par gramme de la composition de mélange final.

L'utilisation de cellules d'au moins une souche de bactéries de la classe des Bacilli selon l'invention est également caractérisée en ce que la composition qui est fabriquée consiste en un aliment complet pour poissons appartenant à la famille des salmonidés.

Selon encore un autre aspect, la composition prophylactique d'additif alimentaire selon l'invention peut être ajoutée ou mélangée à une composition alimentaire conventionnelle adaptée aux différentes phases de croissance du salmonidé considéré. Par exemple, 10 parties en poids de la composition prophylactique selon l'invention peuvent être mélangées à 300 parties en poids d'une composition nutritive commerciale pour poissons de la famille des salmonidés, afin d'obtenir un aliment complet pour salmonidés qui possède un effet prophylactique à l'encontre du syndrome de compression des vertèbres.

De préférence, une composition prophylactique alimentaire selon l'invention est distribuée, en quantités appropriées en fonction du nombre de poissons présents dans le bassin d'élevage considéré, dès l'ouverture de la bouche des larves ou éventuellement encore à partir du stade alevin (10 jours à 10°C après l'éclosion).

Selon l'invention, on met en oeuvre un procédé de traitement prophylactique du syndrome de compression des vertèbres chez les salmonidés, caractérisé en ce qu'il comporte une étape au cours de laquelle une composition d'additif alimentaire telle que définie ci-dessus est distribuée, en des quantités quotidiennes appropriées, dans un bassin d'élevage dans lequel évoluent les salmonidés.

De préférence, la durée pendant laquelle est administrée la composition prophylactique d'additif alimentaire, ou encore la composition alimentaire contenant ladite composition prophylactique d'additif alimentaire est d'au moins trois mois, préférentiellement d'au moins quatre mois, et de manière tout à fait préférée d'au moins cinq mois.

Selon un second mode de réalisation d'une composition prophylactique, l'utilisation selon l'invention est caractérisée en ce que la composition qui est fabriquée consiste en une composition pharmaceutique destinée aux poissons appartenant à la famille des salmonidés.

Pour la fabrication d'une telle composition pharmaceutique, l'homme du métier peut se référer aux techniques conventionnelles de fabrication d'une formulation pharmaceutique en mélangeant un ou plusieurs excipients avec la quantité appropriée de cellules bactériennes d'une souche ou d'un mélange de souches bactériennes de la classe des *Bacilli.*

Par exemple, le support excipient peut consister en du lactose, auquel on ajoute la quantité appropriée de cellules bactériennes.

L'invention est en outre illustrée, sans pour autant être limitée, aux figures et aux exemples suivants.

### FIGURES

La **figure 1** représente des clichés de radiographie aux rayons X de vertèbres d'une colonne vertébrale d'une truite arc-en-ciel non affectée par le syndrome de compression des vertèbres (figure 1A) et un cliché de radiographie aux rayons X de vertèbres mal formées et soudées de la colonne vertébrale d'une truite arc-en-ciel atteinte du syndrome de compression des vertèbres (figure 1B).

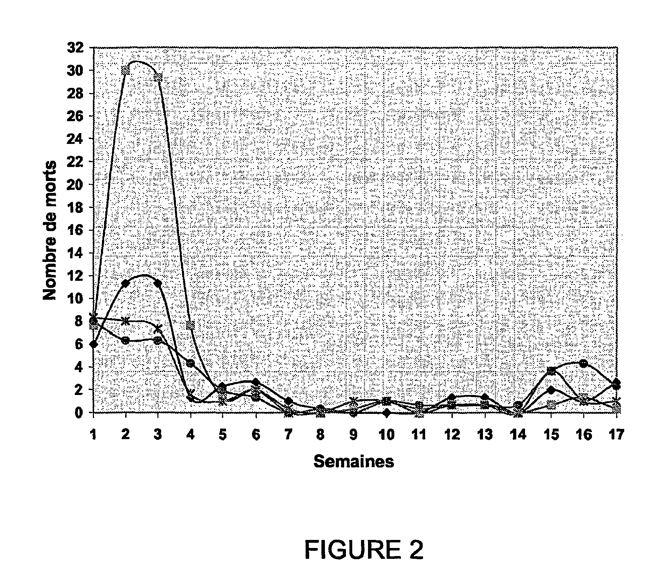

La **figure 2** illustre le taux de mortalité moyen par semaine dans un élevage de truites arc-en-ciel nourries avec (i) un aliment standard (losange plein), (ii) un aliment standard et un supplément antibiotique de florphénicol (carré plein grisé), (iii) un aliment standard et une composition prophylactique nutritionnelle selon l'invention administrées pendant une durée de vingt jours (astérisque) ou de cinq mois (cercle plein).
   En ordonnées, nombre de morts par semaine ; en abscisse, la durée d'élevage, exprimée en nombre de semaines.

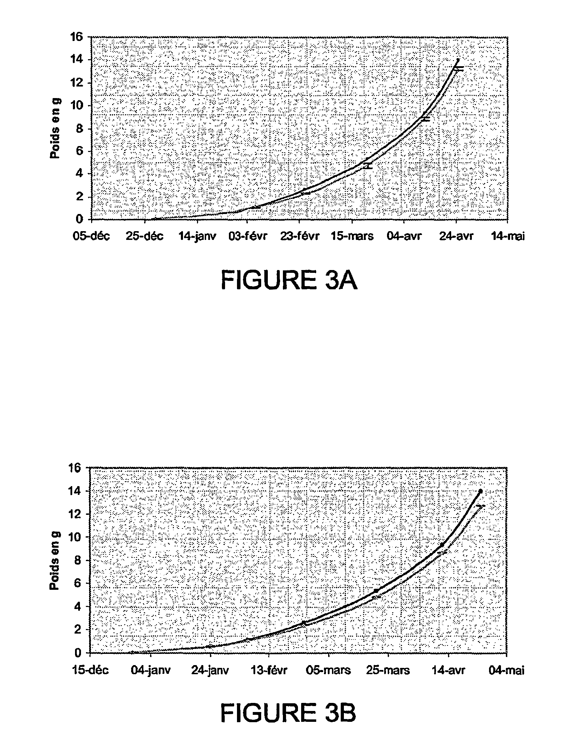

La **figure 3** illustre les résultats comparatifs de la mesure de la croissance des alevins entre des alevins témoins et des alevins ayant reçu une composition d'additif alimentaire conforme à l'invention pendant 20 jours. (Fig.3A) ou pendant 5 mois (Fig.3B).
   En abscisses: temps d'élevage (dates) ; en ordonnées : poids moyen des alevins, exprimé en grammes.

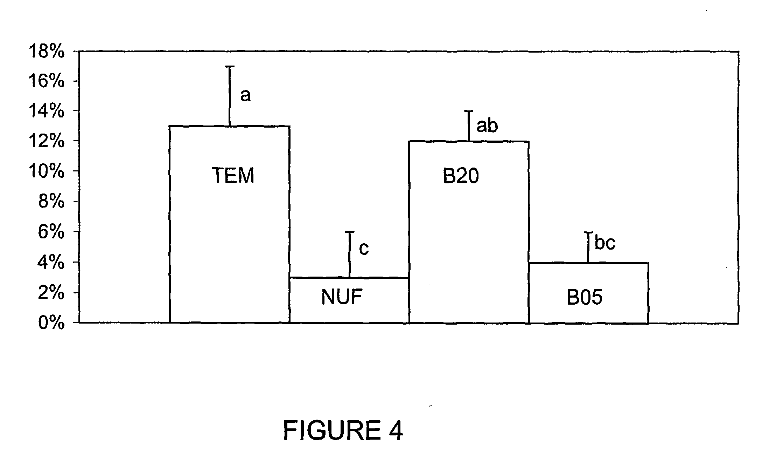

La **figure 4** illustre la fréquence des poissons présentant des compressions de vertèbres, en fonction de leur régime alimentaire. « TEM » = alimentation des poissons avec un aliment standard ; «NUF»= alimentation des poissons avec un aliment standard additionné de l'antibiotique florphénicol ; «B20 » et « B05 » = poissons alimentés avec un aliment standard additionné d'une composition prophylactique contenant des *Lactobacillaceae* d'une souche de *Pediococcus acidilactici,* respectivement pendant une durée de vingt jours (B20) ou de cinq mois (B05).
   En ordonnées, le pourcentage des poissons atteints du syndrome de compression des vertèbres.

### EXEMPLES

### EXEMPLE 1: Procédé de fabrication d'une composition prophylactique nutritionnelle selon l'invention.

### A. MATERIELS ET METHODES

Le procédé de fabrication d'une composition prophylactique alimentaire peut consister en l'ajout des cellules de la ou des souche (s) de la classe des *Bacilli* choisie(s) lors de la fabrication de supports alimentaires commerciaux.

Après la phase de cuisson-extrusion, la base granulée encore chaude est enrobée par brassage avec de l'huile de poisson tiède (30°C). Cette huile peut contenir une suspension particulaire de l'additif alimentaire, comprenant le probiotique et son support, de concentration permettant d'atteindre l'objectif d'ensemencement final.

### B. RESULTATS DE L'ANALYSE BACTERIOLOGIQUE

La composition prophylactique d'additif alimentaire fabriquée conformément à la partie « Matériels et Méthodes » ci-dessus a été analysée pour son contenu en cellules de la souche *Pediococcus acidilactici* vivante. Les résultats sont représentés dans le tableau 1 ci-après.

**TABLEAU 1**

| **Contrôle de l'ensemencement de l'aliment** | | | | | |
|---|---|---|---|---|---|
| **Echantillon** | **Quantité attendue** | | **Quantité mesurée** | | **Différence** |
| Désignation | ufc/g | log | ufc/g | log | log |
| Ecoweaner® n°003 + Bactocell® | 1,50 x 10⁶ | 6,18 | 2,50 x 10⁶ | 6,40 | + 0,222 |
| Ecostart® 15 n°01 + Bactocell® | 1,50 x 10⁶ | 6,18 | 1,30 x 10⁶ | 6,11 | - 0,062 |
| Ecostart® 15 n°02 + Bactocell® | 1,50 x 10⁶ | 6,18 | 1,10 x 10⁶ | 6,04 | - 0,135 |
| Ecostart® 15 n°02 | 1,00 x 10¹ | 1,00 | 1,00 x 10¹ | 1,00 | + 0,000 |

La composition prophylactique ainsi préparée contient bien la quantité de germes viables de *P.acidolactici* désirée, ce qui montre l'efficacité du produit.

### EXEMPLE 2 : Etude comparative d'élevage de truites arc-en-ciel avec une composition prophylactique alimentaire selon l'invention et d'autres compositions alimentaires.

### A. MATERIELS ET METHODES

### A.1 Condition d'élevage des truites arc-en-ciel.

Des truites arc-en-ciel de souche automnale (INRA), en démarrage alimentaire, ont été réparties en six groupes de trois lots distincts, chaque lot comprenant 500 alevins de 10 jours respectivement :
(a) trois lots témoins, nourris exclusivement avec un aliment standard, la composition alimentaire « Ecostart® » commercialisée par la Société BIOMAR SA, lot désigné « TEM » ;
(b) Trois lots nourris avec l'aliment standard Ecostart® et traités par l'antibiotique Florphénicol (spécialité pharmaceutique vétérinaire Nuflor®) pendant dix jours dès la première alimentation à raison d'une quantité quotidienne de Florphénicol de 18 mg par kg de poids vif, ces lots étant désignés « NUF » ;
(c) trois lots nourris avec la composition alimentaire standard supplémentée avec une composition prophylactique nutritionnelle selon l'invention, commercialisée sous la marque Bactocell® par la Société Lallemand, pendant vingt jours dès la première alimentation, ces lots étant désignés « B20 » ; et
(d) trois lots nourris avec la composition alimentaire standard sont supplémentés avec la composition Bactocell® pendant cinq mois dès la première alimentation, ces lots étant désignés « B05 ».

### A.2 Paramètres analysés

Sur les différents lots de poissons ci-dessus, on a contrôlé, respectivement :
- la mortalité des poissons ;
- la croissance des poissons, par prélèvement au hasard de 4 fois 50 poissons dans chacun des lots, à différents temps, et mesure de leur poids total.
- la flore digestive sur l'ensemble des lots (a) à (d) au temps vingt jours et au temps cinq mois après le début de l'essai. Après 12 h de jeûne, trois alevins ont été prélevés dans chaque bassin. Après désinfection de l'abdomen à l'alcool à 70°C, la partie distale du tube digestif a été séparée par dissection en conditions stériles. Après pesée de la prise d'essai, la section du tube digestif a été broyée dans un homogénéiseur en verre contenant 4,5 mL de solution de Ringer. Le broyat a été dilué jusqu'à 1000 fois par dilutions successives au dizième. Les dilutions au dizième et au millième ont été appliquées sur Petrifilm pour le comptage de la flore aérobie (incubation de 7 jours à 11°C).
- la survie des cellules de *Pediococcus acidilactici* dans le tube digestif des poissons au temps vingt jours, trente jours et cinq mois après le début de l'essai. Les broyats de tube digestif préparés selon la technique décrite ci-dessus ont également été utilisé pour le comptage des probiotiques. En fonction des traitements (a) à (d), les dilutions appropriées ont été appliquées sur gélose MRS pour le comptage de *Pediococcus acidilactici* (incubation de 48 h à 45°C), et sur gélose de Sabouraud supplémentée en Chloramphenicol (50 mg L⁻¹), Sulfate de polymyxine B (1,6 mg L⁻¹) et amoxicilline (2,5 mg L⁻¹) pour le comptage de *Saccharomyces cerevisiae* (incubation de 48 h à 30°C).
- la fréquence et de l'intensité du syndrome de compression vertébrale sur les lots (a) à (d), par radiographie ou rayons X sur cinquante poissons de chacun des lots.

### B. RESULTATS

### B.1. Taux de mortalité.

Les résultats d'analyse du taux de mortalité moyen hebdomadaire des poissons appartenant aux lots (a) à (d) sont représentés sur la figure 2.

Les résultats montrent que, dans les premières semaines de l'essai, la mortalité des lots NUF (traitement avec l'antibiotique florphénicol) est particulièrement élevée, cet antibiotique possédant vraisemblablement des propriétés toxiques pour les poissons.

En revanche, aucune différence significative n'est observée sur le taux de mortalité des poissons respectivement dans le lot (a) témoin et dans le lot (d) dont l'alimentation a été supplémentée par une composition à base de *Pediococcus acidilactici* (lots (c) et (d)).

On peut toutefois noter une tendance à une survie meilleure des poissons dans le lot ( d ) traité avec un complément alimentaire, en l'absence d'antibiotique.

### B.2. Analyse de la croissance des alevins

La croissance de l'ensemble des lots de truitelles (a) à (d) était normale. Aucune différence statistiquement significative n'est observée concernant la croissance des alevins selon les différents lots.

Les résultats sont représentés sur la figure 3.

Les résultats de la figure 3 montrent que les alevins ayant reçu une composition d'additif alimentaire conforme à l'invention ont une croissance identique à celle des alevins du lot témoin.

### B.3 Analyse de la flore digestive

Au jour 20 après le début de l'essai, les comptages de germes cultivables totaux sont très variables, et aucune différence statistiquement significative n'est observée selon les lots.

Les quantités de cellules de *Pediococcus acidilactici,* après douze heures de jeune, sont relativement faibles pour les poissons du lot (c) ; elles sont de 2,1 x 10³ ± 1,2 x 10³ cfu par gramme. Dans le lot (c ), dans lesquels la supplémentation nutritionnelle a été arrêtée au jour 20, plus aucune cellule de *Pediococcus acidilactici* (lot (c)) n'est retrouvée. Il n'y a donc pas de persistance des germes dans le tube digestif des poissons, après l'arrêt du traitement par le supplément nutritionnel.

Au temps cinq mois après le début de l'essai, on a observé une différence significative dans les comptages de germes cultivables totaux, respectivement :
- lot (a) témoin :1,4 x 10⁷ ± 0,8 x 10⁷ cfu par gramme ;
- lot *Pediococcus* (d) : 6,4 x 10⁴ ± 2,4 x 10⁴ cfu par gramme.

Aucune cellule de *Pediococcus acidilactici* n'a été retrouvée dans le tube digestif des poissons après vingt quatre heures de jeune, quel que soit le lot considéré. Cette observation confirme la non implantation des probiotiques dans le tube digestif.

Ces résultats montrent aussi que la composition prophylactique de supplément nutritionnel selon l'invention exerce un effet régulateur sur la flore digestive des poissons.

### B.4. Fréquence d'apparition du syndrome de compression des vertèbres

La fréquence d'apparition du syndrome de compression des vertèbres a été analysée dans chacun des lots (a) à (d) . Les résultats sont représentés sur la figure 4.

Comme le montrent les résultats de la figure 4, le traitement des poissons par la composition prophylactique de supplément nutritionnel selon l'invention, à base de cellules de *lactobacilles Pediococcus acidilactici,* et après un traitement d'une durée de cinq mois, réduit considérablement la fréquence des poissons atteints du syndrome de compression des vertèbres, réduction qui est du même ordre que celle observée avec l'antibiotique florphénicol.

Les résultats ci-dessus mettent en évidence l'intérêt de l'utilisation de cellules d'au moins une souche de bactéries de la classe des *Bacilli* pour la fabrication d'une composition destinée à prévenir le syndrome de compression des vertèbres chez les salmonidés, en particulier chez la truite arc-en-ciel.

Ainsi, l'utilisation, comme additif alimentaire, d'une composition prophylactique selon l'invention apparaît bien adaptée à la réduction drastique de l'incidence du syndrome de compression des vertèbres chez les salmonidés, sans entraîner les nombreux inconvénients liés à l'utilisation d'un composé antibiotique. Une telle composition utilisée conformément à l'invention est donc bien adaptée au cadre sanitaire actuel de l'élevage piscicole et est bien moins risquée pour le cheptel que l'utilisation d'antibiotiques à titre préventif.

En outre, une composition prophylactique selon l'invention se caractérise par sa facilité de mise en oeuvre et son coût réduit. La rentabilité des élevages de salmonidés dans des piscicultures est accrue.

## Revendications

1. Utilisation de cellules d'au moins une souche de bactéries de la classe des *Bacilli* pour la fabrication d'une composition destinée à prévenir le syndrome de compression des vertèbres chez un poisson appartenant à la famille des salmonidés.

2. Utilisation selon la revendication 1, **caractérisée en ce que** ladite composition comprend de 10³ à 10⁸ unités formant des colonies (cfu) de ladite bactérie de la classe des *Bacilli,* par gramme de la composition.

3. Utilisation selon la revendication 2, **caractérisée en ce que** ladite composition comprend de 10⁴ à 10⁷ unités formant des colonies (cfu) de ladite bactérie de la classe des *Bacilli,* par gramme de la composition.

4. Utilisation selon l'une des revendications 1 à 3, **caractérisée en ce que** la ou les souche(s) de bactérie(s) de la classe des *Bacilli* est (sont) choisie(s) parmi les bactéries des familles *Lactobacillaceae, Carnobacteriaceae, Enterococcaceae, Leuconostocaceae* et *Streptococcacea.*

5. Utilisation selon l'une des revendications 1 à 3, **caractérisée en ce que** la ou les souche(s) de bactérie(s) de la classe des *Bacilli* est (sont) choisie(s) parmi les souches *Bacillus subtilis, Bacillus cereus, Bacillus megaterium, Bacillus polymyxa, Bacillus licheniformis, Lactobacillus rhamnosus, Lactobacillus plantarum, Lactobacillus casei, Lactobacillus acidophilus , Lactobacillus johnsonii, Lactobacillus helveticus, Lactobacillus bulgaricus, Lactobacillus fructivorans, Lactobacillus brevis, Pediococcus acidilactici , Pediococc us pentosaceus, Carnobacterium divergens, Carnobacterium inhibens , Enterococcus faecium, Leuconostoc cremoris , Weissella hellenica Streptococcus thermophilus, Lactococcus lactis.*

6. Utilisation selon l'une des revendications 1 à 5, **caractérisée en ce que** le poisson appartenant à la famille des salmonidés est choisi parmi les saumons, les truites, les ombles, l'ombre et les corégones.

7. Utilisation selon l'une des revendications 1 à 6, **caractérisée en ce que** ladite composition consiste en une composition d'additif alimentaire destinée aux poissons appartenant à la famille des salmonidés.

8. Utilisation selon la revendication 7, **caractérisée en ce que** ladite composition consiste en un aliment complet pour poissons appartenant à la famille des salmonidés.

9. Utilisation selon l'une des revendications 1 à 6, **caractérisée en ce que** ladite composition consiste en une composition pharmaceutique destinée aux poissons appartenant à la famille des salmonidés.

## Claims

1. Use of cells of at least one bacterium strain of the *Bacilli* class for preparing a composition intended for preventing the vertebral compression syndrome in a fish belonging to the salmonid family.

2. Use according to claim 1, **characterized in that** said composition comprises 10³ to 10⁸ colony forming units (cfu) of said bacterium of the *Bacilli* class per gram of the composition.

3. Use according to claim 2, **characterized in that** said composition comprises 10⁴ to 10⁷ colony forming units (cfu) of said bacterium of the *Bacilli* class per gram of the composition.

4. Use according to one of claims 1 to 3, **characterized in that** said bacterium strain(s) of the *Bacilli* class is (are) selected amongst bacteria of the *Lactobacillaceae, Carnobacteriaceae, Enterococcaceae, Leuconostocaceae* and *Streptococcacea* families.

5. Use according to one of claims 1 to 3, **characterized in that** the bacterium strain(s) of the *Bacilli* class is (are) selected amongst the *Bacillus subtilis, Bacillus cereus, Bacillus megaterium, Bacillus polymyxa, Bacillus licheniformis, Lactobacillus rhamnosus, Lactobacillus plantarum, Lactobacillus casei, Lactobacillus acidophilus, Lactobacillus johnsonii, Lactobacillus helveticus, Lactobacillus bulgaricus, Lactobacillus fructivorans, Lactobacillus brevis, Pediococcus acidilactici, Pediococcus pentosaceus, Carnobacterium divergens, Carnobacterium inhibens, Enterococcus faecium, Leuconostoc cremoris, Weissella hellenica, Streptococcus thermophilus, Lactococcus lactis* strains.

6. Use according to one of claims 1 to 5, **characterized in that** the fish belonging to the salmonid family is selected amongst salmons, trouts, char fishes, graylings and coregons.

7. Use according to one of claims 1 to 6, **characterized in that** said composition consists in a food additive composition for fish belonging to the salmonid family.

8. Use according to claim 7, **characterized in that** said composition consists in a whole foodstuff for fish belonging to the salmonid family.

9. Use according to one of claims 1 to 6, **characterized in that** said composition consists in a pharmaceutical composition for fish belonging to the salmonid family.

## Patentansprüche

1. Verwendung von Zellen wenigstens eines Bakterienstamms aus der Klasse Bacilli zur Herstellung einer Zusammensetzung zur Prävention des VCCS (vertebral column compression syndrome) bei einem Fisch, der zur Familie der Salmoniden gehört.

2. Verwendung gemäß Anspruch 1, **dadurch gekennzeichnet, dass** die Zusammensetzung 10³ bis 10⁸ koloniebildende Einheiten (KBE) des Bakteriums der Klasse Bacilli pro Gramm der Zusammensetzung umfasst.

3. Verwendung gemäß Anspruch 2, **dadurch gekennzeichnet, dass** die Zusammensetzung 10⁴ bis 10⁷ koloniebildende Einheiten (KBE) des Bakteriums der Klasse Bacilli pro Gramm der Zusammensetzung umfasst.

4. Verwendung gemäß einem der Ansprüche 1 bis 3, **dadurch gekennzeichnet, dass** der bzw. die Bakterienstämme der Klasse Bacilli aus Bakterien der Familien Lactobacillaceae, Carnobacteriaceae, Enterococcaceae, Leuconostocaceae und Streptococcaceae ausgewählt ist/sind.

5. Verwendung gemäß einem der Ansprüche 1 bis 3, **dadurch gekennzeichnet, dass** der bzw. die Bakterienstämme der Klasse Bacilli aus den Stämmen *Bacillus subtilis, Bacillus cereus, Bacillus megaterium, Bacillus polymyxa, Bacillus licheniformis, Lactobacillus rhamnosus, Lactobacillus plantarum, Lactobacillus casei, Lactobacillus acidophilus, Lactobacillus johnsonii, Lactobacillus helveticus, Lactobacillus bulgaricus, Lactobacillus fructivorans, Lactobacillus brevis, Pediococcus acidilactici, Pediococcus pentosaceus, Carnobacterium divergens, Carnobacterium inhibens, Enterococcus faecium, Leuconostoc cremoris, Weissella hellenica, Streptococcus thermophilus* und *Lactococcus lactis* ausgewählt ist/sind.

6. Verwendung gemäß einem der Ansprüche 1 bis 5, **dadurch gekennzeichnet, dass** der Fisch, der zur Familie der Salmoniden gehört, aus Lachsen, Forellen, Saiblingen, der Äsche und Renken ausgewählt ist.

7. Verwendung gemäß einem der Ansprüche 1 bis 6, **dadurch gekennzeichnet, dass** die Zusammensetzung aus einer Nahrungszusatzzusammensetzung für Fische, die zur Familie der Salmoniden gehören, besteht.

8. Verwendung gemäß Anspruch 7, **dadurch gekennzeichnet, dass** die Zusammensetzung aus einer Vollnahrung für Fische, die zur Familie der Salmoniden gehören, besteht.

9. Verwendung gemäß einem der Ansprüche 1 bis 6, **dadurch gekennzeichnet, dass** die Zusammensetzung aus einer pharmazeutischen Zusammensetzung für Fische, die zur Familie der Salmoniden gehören, besteht.
